# EUROPÄISCHE PATENTANMELDUNG

(11) **EP 4 706 549 A1**
(43) Veröffentlichungstag der Anmeldung: **11.03.2026**
(21) Anmeldenummer: 25200257.1
(22) Anmeldetag: 04.09.2025
(51) Int. Cl.: A61B 17/00, A61B 1/00, A61B 90/00, A61B 90/57, F16B 2/00, F16B 2/22, F16B 7/04, F16L 3/13, A61M 25/02, A61B 17/16

(54) **MEDIZINISCHER HALTECLIP, MEDIZINISCHE VORRICHTUNG ZUR AUFNAHME EINES MEDIZINISCHEN WERKZEUGS UND VERFAHREN ZUM WERKZEUGWECHSEL AN EINER MEDIZINISCHEN VORRICHTUNG**

(30) Priorität: 04.09.2024 DE 102024125353
(71) Anmelder: Aesculap AG, 78532 Tuttlingen (DE)
(72) Erfinder: LENZENHUBER, Frederick, 78532 Tuttlingen (DE); BAUER, Stephan, 78576 Emmingen (DE)
(74) Vertreter: Winter, Brandl - Partnerschaft mbB

(57) **Zusammenfassung**

Die vorliegende Offenbarung betrifft einen medizinischen Halteclip (1) zur lösbaren Montage an einem medizinischen Handstück (3) einer medizinischen Vorrichtung (4) mit
- einem Montagebereich (5), der zwei Clipflügel (6, 7) aufweist, die gemeinsam eine C- oder U-Form ausbilden und die, in einer Halteclip-Querschnittsebene (Q) gesehen, elastisch längs einer ersten Richtung (R1) biegbar sind, um die C- oder U-Form für eine Clipmontage des Halteclips (1) auf oder an dem Medizinprodukt (2) aufzuweiten und
- einem Rast- oder Klemmbereich (8), der einen einstückig mit dem Montagebereich (5) ausgeformten oder an diesem befestigten Klemmflügel (10) aufweist und dafür vorgesehen und ausgebildet ist, einen Schlauch oder ein Rohr (9) vorzugsweise parallel zum Medizinprodukt (2) klemmend sowie lösbar aufzunehmen.

Der medizinische Halteclip (1) ist verbessert, indem der Klemmflügel (10) zwischen sich und dem Rast- oder Klemmbereich (8) oder dem Montagebereich (5) einen einseitig offenen Aufnahmespalt (11) ausbildet und für ein Aufweiten des Aufnahmespalts (11), in der Halteclip-Querschnittsebene (Q) gesehen, längs einer zweiten Richtung (R2) elastisch biegbar ist, welche in einem Winkel (α) ungleich 0° zur ersten Richtung (R1) steht. Ferner betrifft die vorliegende Offenbarung eine medizinische Vorrichtung (4) zur Aufnahme eines medizinischen Werkzeugs (17) und ein Verfahren zum Werkzeugwechsel an einer medizinischen Vorrichtung (4).

## Beschreibung

Die vorliegende Offenbarung betrifft einen medizinischen Halteclip gemäß dem Oberbegriff des Patentanspruchs 1, sowie eine medizinische Vorrichtung zur Aufnahme eines medizinischen Werkzeugs und ein Verfahren zum Werkzeugwechsel an einer medizinischen Vorrichtung.

### Stand der Technik

Aus dem Stand der Technik sind Medizinprodukte, medizinische Vorrichtungen, insbesondere chirurgische Vorrichtungen in Form von Handgeräten wie Handbohrer oder Handfräser bekannt, die eine Zuführung von Spülflüssigkeit durch einen außen am Handgerät angebrachten Schlauch bzw. ein hier angebrachtes Rohr oder eine Zuführung von Spülflüssigkeit durch ein fest innen im Handgerät geführten Durchgang bereitstellen. Insbesondere wird zumeist eine sterile Spülgarnitur (Beutel/Schlauchkombination) an den Schlauch oder das Rohr angeschlossen bzw. gekoppelt, um eine sterile Spülflüssigkeit durch den Schlauch oder das Rohr zu spülen. Die sterile Flüssigkeit muss hier den sterilen Schlauch verlassen und wird dann durch den an einem Handstück der medizinischen Vorrichtung geführten Schlauch oder das Rohr zum Werkzeug/Situs geleitet. Der Schlauch oder das Rohr haben wegen der geringen Spülmenge bzw. dem geringen Spüldurchfluss meist einen kleinen Innendurchmesser. Es sind gewendelte / gebogene Rohre bekannt, welche auf das Handstück der medizinischen Vorrichtung aufgeschoben werden können und welche ohne weitere Bauteile an der medizinischen Vorrichtung halten.

Es sind weiterhin medizinische Halteclips bekannt, mittels welchen der Schlauch oder das Rohr von außen an der medizinischen Vorrichtung, insbesondere dessen Handstück, angebracht ist. Solche medizinischen Halteclips sind zur lösbaren Montage an dem Medizinprodukt insbesondere an dem medizinischen Handstück der medizinischen Vorrichtung ausgebildet und weisen eine Montagebereich auf, der zwei Clipflügel aufweist, die gemeinsam eine C- oder U-Form ausbilden und die, in einer Halteclip-Querschnittsebene gesehen, elastisch längs einer ersten Richtung biegbar sind, um die C- oder U-Form für eine Clipmontage des Halteclips auf oder an dem Medizinprodukt aufzuweiten. Solche medizinischen Halteclips weisen weiterhin einen Rast- oder Klemmbereich auf, der dafür vorgesehen und ausgebildet ist, einen Schlauch oder ein Rohr vorzugsweise parallel zum Medizinprodukt klemmend sowie lösbar aufzunehmen.

Es sind Halteclips bekannt, die in einer Halteclip-Querschnittsebene gesehen zwei Klemmflügel aufweisen, zwischen denen sich ein einseitig offener, in radialer Richtung ausgerichteter Aufnahmespalt ausbildet, dessen äußere Öffnung parallel zu einer Tangentialen zu den Clipflügeln liegt. Diese beiden Klemmflügel sind dann jeweils in der Halteclip-Querschnittsebene gesehen, längs einer Richtung elastisch biegbar, die parallel zur ersten Richtung liegt.

Die Richtung, in welcher die Klemmflügel sich verbiegen, wird dabei wie folgt bestimmt. Es wird die Situation betrachtet, in welcher kein Schlauch oder Rohr im Klemmbereich aufgenommen ist. Aus dieser Konstruktionslage heraus beginnen sich die Klemmflügel zu verbiegen, wenn der Schlauch oder das Rohr von oben durch die Öffnung des Aufnahmespalts geführt wird. Das Verbiegen beginnt, wenn der erste Kontakt zwischen Schlauch oder Rohr und den Klemmflügeln erzeugt wird. Mit der Richtung, in welcher die Klemmflügel sich verbiegen, ist die Richtung gemeint, in welcher sich der jeweilige Klemmflügel, insbesondere das auskragende Ende der neutralen Faser des jeweiligen Klemmflügels, zu Beginn des Verbiegens verbiegt, wobei diese Richtung eben wie oben beschrieben parallel zur ersten Richtung liegt. Im weiteren Verlauf des Verbiegens ändert sich die Richtung der Verbiegung entlang einer Kurve, was aber für die Definition der Richtung bzw. deren Winkel im Sinne dieses Anmeldetextes irrelevant ist.

Die bekannten Halteclips weisen aber zur Sicherstellung der Funktionalität und der Stabilität große Abmaße auf, wobei insbesondere der Schlauch oder das Rohr nur mit vergleichsweise großem Abstand zur medizinischen Vorrichtung mit dieser mittels des Halteclips verbindbar ist. Dann ist aber das Bewegen der medizinischen Vorrichtung in den unter Umständen sehr beengten Operationsgebieten durch den Halteclip und den Schlauch oder das Rohr limitiert und bestimmte Operationen können dann nicht durchgeführt werden.

Zur Befestigung der Schläuche oder Rohre an der medizinischen Vorrichtung sind weiterhin Kunststoffträger mit zusätzlichem Klebeelement bekannt, welche aber aufwendig in der Herstellung und schwierig im Handling sind. Weiterhin sind Kleberückstände nach Gebrauch, wenn überhaupt, nur mit hohem Aufwand wieder entfernbar.

Es sind weiterhin medizinische Halteclips bekannt, welche während ihrer Herstellung im Spritzgussverfahren um den Schlauch oder das Rohr herumgespritzt werden, wobei der Schlauch oder das Rohr ein sogenanntes Einlegeteil bilden. Der Aufwand zur Herstellung einer solchen Konstruktion ist aber hoch. Weiterhin muss beachtet werden, dass keine Prozesshilfsstoffe in den Schlauch oder das Rohr gelangen.

Es sind gelochte medizinische Halteclips bekannt, bei welchen der Schlauch oder das Rohr in dessen Längsrichtung in das Loch / die Bohrung zur Verbindung mit dem jeweiligen Halteclip eingeführt werden muss. Dabei besteht die Gefahr, dass Abrieb in den Schlauch oder das Rohr gelangt und bei der späteren Operation in den Patienten verschleppt wird.

### Kurzbeschreibung der Offenbarung

Es ist also die Aufgabe der Offenbarung, die Nachteile aus dem Stand der Technik zu vermeiden oder wenigstens zu mildern.

Diese Aufgabe wird zunächst durch einen medizinischen Halteclip gemäß Anspruch 1 gelöst.

Der funktionale Grundgedanke der vorliegenden Offenbarung besteht im Prinzip darin, den Halteclip in einer vordefinierten Auf-/Ancliprichtung mit dem Medizinprodukt in Montageeingriff zu bringen und ein weiteres Rohr oder einen Schlauch in einer vordefinierten Klemmrichtung an den Halteclip anzuklemmen, die in einem Winkel ungleich 0° zu der Auf-/Ancliprichtung steht. Konstruktiv läßt sich dieses Funktionsprinzip folgendermaßen umsetzen:
Ein Aspekt der Offenbarung liegt zunächst im Wesentlichen darin, dass der Rast- oder Klemmbereich des Halteclips für den Schlauch oder das Rohr einen vorzugsweise einstückig mit dem Montagebereich zum Auf-/Anclipen des Halteclips am Medizinprodukt ausgeformten oder an diesem befestigten Klemmflügel aufweist, der zwischen sich und dem Rast- oder Klemmbereich oder zwischen sich und dem Montagebereich einen einseitig offenen Aufnahmespalt ausbildet, wobei der Klemmflügel für ein Aufweiten des (Rohr-/Schlauch-)Aufnahmespalts, in der Halteclip-Querschnittsebene gesehen, längs einer (zweiten) Richtung elastisch biegbar ist, welche in einem Winkel ungleich 0° zu einer ersten Richtung steht, in der sich Clipflügel des Montagebereichs beim Auf-/Anclipen des Halteclips am Medizinprodukt aufweiten. Auf diese Weise wird ein unbeabsichtigtes Abstreifen eines eingeklemmten Rohrs/Schlauchs beispielsweise bei der Handhabung des Medizinprodukts oder beim Aufclipen des Halteclips an/auf das Medizinprodukt vermieden oder zumindest erschwert, da die Klemmrichtung für das Rohr oder den Schlauch unterschiedlich zur Cliprichtung ist.

Zusätzlich kann der Aufnahmespalt seine Spalt-Längsrichtung verändern, beispielsweise durch Ausbildung eines hakenförmigen Längsknicks, wodurch ein unbeabsichtigtes Herausdrücken des Schlauchs/Rohrs aus dem Aufnahmespalt weiter erschwert ist.

Der Schlauch oder das Rohr sind vorteilhafterweise in einer Richtung senkrecht zu deren Längsachse seitlich in den Aufnahmespalt einführbar. Vorzugsweise weist der Winkel zwischen der ersten und der zweiten Richtung in der Halteclip-Querschnittsebene Werte größer 0° und kleiner oder gleich 90° auf. Insbesondere weist der Winkel zwischen der ersten und der zweiten Richtung in der Halteclip-Querschnittsebene Werte zwischen 75° und 90°, besonders bevorzugt von 90°, auf. Ein derartig ausgeführter Halteclip ist besonders kompakt ausführbar. Insbesondere ist eine dem Montagebereich abgewandte Außenseite des Klemmbereiches bzw. des Klemmflügels mit minimalem Abstand zu einem Innenseitenabschnitt der Clipflügel ausbildbar. Mit dem derartigen Halteclip ist weiterhin ein besonders geringer Abstand zwischen dem dann mit dem Halteclip verbundenen Medizinprodukt und dem dann mit dem Halteclip verbundenen Schlauch oder Rohr realisierbar. Das Bewegen der medizinischen Vorrichtung in den unter Umständen sehr beengten Operationsgebieten ist durch den derartigen Halteclip und dem damit verbundenen Schlauch oder Rohr nur minimal limitiert, so dass eine Vielzahl von unterschiedlichen Operationen durchgeführt werden können.

Vorzugsweise weist der Rast- oder Klemmbereich genau einen Klemmflügel auf.

Vorzugsweise definieren die Clipflügel einen Innenseitenabschnitt, welcher dafür vorgesehen ist, zumindest teilweise / abschnittsweise, mit dem Medizinprodukt in vorzugsweise linienförmigen Klemmeingriff zu kommen.

Vorzugsweise definieren die Clipflügel einen, von dem Innenseitenabschnitt abgewandten Außenseitenabschnitt.

Eine Oberflächennormale des Innenseitenabschnitts zeigt vorzugsweise nach (radial) innen. Eine Oberflächennormale des Außenseitenabschnitts zeigt vorzugsweise nach (radial) außen.

Der Außenseitenabschnitt ist vorzugsweise dafür vorgesehen, zusammen mit dem Klemmflügel den Aufnahmespalt zu bilden.

Der Innenseitenabschnitt kann einen Abschnitt aufweisen, der dafür vorgesehen ist nicht mit dem Medizinprodukt in Klemmeingriff zu kommen. Der Abschnitt ist vorzugsweise zwischen Abschnitten des Innenseitenabschnitts ausgebildet, die dafür vorgesehen sind, mit dem Medizinprodukt in Klemmeingriff zu kommen. Der Abschnitt des Innenseitenabschnitts, der dafür vorgesehen ist nicht mit dem Medizinprodukt in Klemmeingriff zu kommen, ist vorzugsweise dafür vorgesehen, zusammen mit dem Klemmflügel den Aufnahmespalt zu bilden.

Der Innenseitenabschnitt kann dafür vorgesehen sein, zusammen mit dem Klemmflügel den Aufnahmespalt zu bilden.

Der Außenseitenabschnitt und der Innenseitenabschnitt können dafür vorgesehen sein, zusammen mit dem Klemmflügel den Aufnahmespalt zu bilden.

Der Klemmflügel ist vorzugsweise im Wesentlichen L-förmig ausgebildet. Dann ist ein kurzer Schenkel des L-förmigen Klemmflügels einstückig mit dem Montagebereich ausgeformt oder an diesem befestigt bzw. grenzt an diesen an und mittels eines langen Schenkels des L-förmigen Klemmflügels ist das auskragende Ende des Klemmflügels gebildet. Dadurch kann die vorstehend angedeutete Richtungsänderung im Aufnahmespalt erreicht werden.

Gemäß einer vorteilhaften Ausführungsform des medizinischen Halteclips erstreckt sich der Aufnahmespalt zumindest längsabschnittsweise im Wesentlichen tangential zur C- oder U-Form der Clipflügel des Montagebereichs. Insbesondere steht die zweite Richtung in der Halteclip-Querschnittsebene gesehen dann senkrecht zur ersten Richtung. Durch diese tangentiale Ausrichtung des Aufnahmespaltes ist der für den Halteclip benötigte Platzbedarf weiter reduzierbar.

Nach einer weiteren vorteilhaften Ausführungsform des medizinischen Halteclips ist der Aufnahmespalt, in Auskragrichtung des Klemmflügels gesehen, geradlinig, mäanderförmig, gekrümmt oder mit wenigstens einer knickförmigen Abbiegung ausgebildet.

Eine geradlinige Ausführung ist besonders einfach ausbildbar und führt zu einer besonders kostengünstigen Herstellung des Halteclips. Der mäanderförmige oder gekrümmte oder mit wenigstens einer knickförmigen Abbiegung ausgebildete Aufnahmespalt führt zu einer erweiterten Funktionalität des Halteclips, wobei insbesondere ein Risiko des unerwünschten Herausgleitens des Schlauches oder Rohres minimiert zumindest aber verringert ist.

Es kann vorteilhaft sein, wenn der Außenseitenabschnitt der Clipflügel (bzw. des Montageabschnitts) und/oder eine dem Außenseitenabschnitt zugewandte Seite des Klemmflügels, in Auskragrichtung des Klemmflügels gesehen, gewellt oder gerippt ist, um zumindest eine, vorzugsweise eine Mehrzahl von, in Auskragrichtung des Klemmflügels beabstandete einzelne Rastpositionen innerhalb des Aufnahmespalts zu definieren.

Somit sind unterschiedliche Positionen des Schlauches oder Rohres zu einem Ende des Medizinproduktes, insbesondere eines mit dem Medizinprodukt aufgenommenen medizinischen Werkzeugs, realisierbar. Durch entsprechende Ausführung der Wellen oder Rippen sind Schläuche oder Rohre unterschiedlichen Durchmessers einklemmbar und/oder Schläuche oder Rohre gleichen Durchmessers verschieden stark einklemmbar.

Bevorzugterweise hat der Aufnahmespalt einen an die in Auskragrichtung des Klemmflügels gesehen, innerste Rastposition unmittelbar angrenzenden Zufuhrabschnitt, durch welchen der Schlauch oder das Rohr zur innersten Rastposition hinführbar ist und der sich, in der Halteclip-Querschnittsebene gesehen, längs einer dritten Richtung erstreckt, die in einem Winkel von 65° bis 115°, vorzugsweise von 80° bis 100°, insbesondere von 90°, zu einer Senkrechten auf die erste Richtung steht.

Diese innerste Rastposition ist insbesondere die Rastposition, in welcher der Schlauch oder das Rohr während einer Operation angeordnet wird. Insbesondere steht die dritte Richtung im Wesentlichen senkrecht zur zweiten Richtung.

Weiter bevorzugt ist ein Abstand zwischen dem Innenseitenabschnitt und dem Außenseitenabschnitt der Clipflügel in radialer Richtung zur Rastposition hin maximal so groß wie eine Dicke der Clipflügel benachbart des Rast- oder Klemmbereichs. Dies ist insbesondere dadurch ermöglicht, dass der kurze Schenkel des dann L-förmigen Klemmflügels in Umfangrichtung der C- oder U-Form der Clipflügel gesehen neben und mit einem bestimmten Abstand zu der Rastposition angeordnet und/oder ausgebildet ist. Somit ist die Kompaktheit des Halteclips weiter gesteigert.

Insbesondere weist der Aufnahmespalt zwischen seiner äußeren Öffnung und der innersten Rastposition eine Vor-Rastposition auf, in welcher der Schlauch oder das Rohr anordenbar, insbesondere einklemmbar ist. Vorzugsweise sind Schläuche oder Rohre gleichen Durchmessers in der innersten Rastposition mit größerer Klemmkraft einklemmbar als in der Vor-Rastposition.

Nach einer weiteren vorteilhaften Ausführungsform des medizinischen Halteclips ist die Vor-Rastposition über einen Aufnahmespaltabschnitt größter Krümmung, vorzugsweise mit knickförmiger Abbiegung an die Aufnahmespaltöffnung angeschlossen. Dieser Aufnahmespaltabschnitt größter Krümmung, vorzugsweise mit knickförmiger Abbiegung ist vorzugsweise mittels einer Erhebung an dem hier angeordneten Clipflügel ausgebildet. Insbesondere ist der hier, insbesondere benachbart der äußeren Öffnung des Aufnahmespaltes angeordnete Clipflügel mit bereichsweise vergrößerter Dicke, eben im Bereich der Erhebung, ausgeführt.

Ferner wird die Aufgabe gelöst durch eine medizinische Vorrichtung zur Aufnahme eines medizinischen Werkzeugs mit einem Handstück, mit welchem die medizinische Vorrichtung von einem Mediziner führbar ist und mit zumindest einem im Vorhinein beschriebenen medizinischen Halteclip, mittels welchem ein Schlauch oder ein Rohr lösbar mit der medizinischen Vorrichtung, insbesondere dessen Handstück, verbindbar und/oder verbunden ist.

Eine derartig ausgeführte, medizinische Vorrichtung ist besonders kompakt ausführbar. Insbesondere durch Integration des beschriebenen Halteclips ist nämlich ein besonders geringer Abstand zwischen der dann mit dem Halteclip verbundenen medizinischen Vorrichtung und dem dann mit dem Halteclip verbundenen Schlauch oder Rohr realisierbar. Das Bewegen der medizinischen Vorrichtung in den unter Umständen sehr beengten Operationsgebieten ist durch den derartigen Halteclip und dem damit verbundenen Schlauch oder Rohr nur minimal limitiert, so dass eine Vielzahl von unterschiedlichen Operationen mit der derartigen medizinischen Vorrichtung durchgeführt werden können. Mittels der medizinischen Vorrichtung sind verschiedene Werkzeuge zur Durchführung unterschiedlicher operativer Aufgaben aufnehmbar.

Weiterhin wird die Aufgabe gelöst durch ein Verfahren zum Werkzeugwechsel an einer im Vorhinein beschriebenen medizinischen Vorrichtung, in welchem das Handstück mit dem daran lösbar verbundenen medizinischen Halteclip zum Freigeben oder Arretieren des medizinischen Werkzeuges um die Längsachse des Handstückes gedreht wird, währenddessen der Schlauch oder das Rohr in dem medizinischen Halteclip eingeklemmt ist und eingeklemmt bleibt.

Der Werkzeugwechsel ist somit in besonders wenigen Schritten durchführbar, insbesondere ohne den Schlauch oder das Rohr neu montieren zu müssen. Der Halteclip wird vorzugsweise so mit dem Handstück verbunden, dass die äußere Öffnung des Aufnahmespaltes beim Drehen des Handstückes in seine verdrehte Stellung, in welcher das Werkzeug freigegeben ist, von der Rastposition gesehen in Drehrichtung liegt. Dann ist ein Herausgleiten des Schlauches oder des Rohres aus dem Aufnahmespalt besonders sicher vermeidbar.

Bevorzugterweise wird der Schlauch oder das Rohr beim Drehen des Handstückes in seine verdrehte Stellung, in welcher das Werkzeug freigegeben ist, zu einer Helix verformt.

Dabei ist der Schlauch oder das Rohr mit einem weiteren, sogenannten Vor-Halteclip in einem Bereich neben dem Handstück mit der medizinischen Vorrichtung verbunden. Die Relativposition des Vor-Halteclips zur medizinischen Vorrichtung wird während des Drehens nicht verändert.

### Kurzbeschreibung der Figuren

Im Nachfolgenden werden bevorzugte Ausführungsformen unter Bezugnahme auf die anliegenden Figuren näher dargestellt.
Fig. 1 zeigt schematisch eine erste Ausführungsform eines medizinischen Halteclips und eine erste Ausführungsform einer medizinischen Vorrichtung mit dem an der medizinischen Vorrichtung montierten Halteclip in einer dreidimensionalen Ansicht.
Fig. 2 zeigt schematisch die erste Ausführungsform des medizinischen Halteclips in einer Seitenansicht zur Halteclip-Querschnittsebene hin.
Fig. 3a zeigt schematisch eine zweite Ausführungsform des medizinischen Halteclips in einer Seitenansicht zur Halteclip-Querschnittsebene hin.
Fig. 3b zeigt schematisch die zweite Ausführungsform des medizinischen Halteclips mit einem in einer Vor-Rastposition angeordneten Schlauch oder Rohr in einer Seitenansicht zur Halteclip-Querschnittsebene hin.
Fig. 3c zeigt schematisch die zweite Ausführungsform des medizinischen Halteclips mit einem in einer innersten Rastposition angeordneten Schlauch oder Rohr in einer Seitenansicht zur Halteclip-Querschnittsebene hin.
Fig. 4 zeigt schematisch eine dritte Ausführungsform des medizinischen Halteclips in einer Seitenansicht zur Halteclip-Querschnittsebene hin.
Fig. 5a zeigt schematisch die erste Ausführungsform des medizinischen Halteclips und eine zweite Ausführungsform einer medizinischen Vorrichtung mit dem an der medizinischen Vorrichtung montierten Halteclip in einer dreidimensionalen Ansicht.
Fig. 5b zeigt schematisch die erste Ausführungsform des medizinischen Halteclips und eine zweite Ausführungsform einer medizinischen Vorrichtung mit dem an der medizinischen Vorrichtung montierten Halteclip in einer dreidimensionalen Ansicht, aber mit einem Handstück der medizinischen Vorrichtung in einer gegenüber der in Fig.5a gezeigten, verdrehten Stellung.
Fig. 6a zeigt schematisch die zweite Ausführungsform des medizinischen Halteclips mit einem in der innersten Rastposition angeordneten Schlauch oder Rohr in einer dreidimensionalen Ansicht.
Fig. 6b zeigt schematisch die zweite Ausführungsform des medizinischen Halteclips mit einem schräg zur innersten Rastposition und schräg zur Vor-Rastposition angeordneten Schlauch oder Rohr in einer dreidimensionalen Ansicht.

### Beschreibung bevorzugter Ausführungsbeispiele

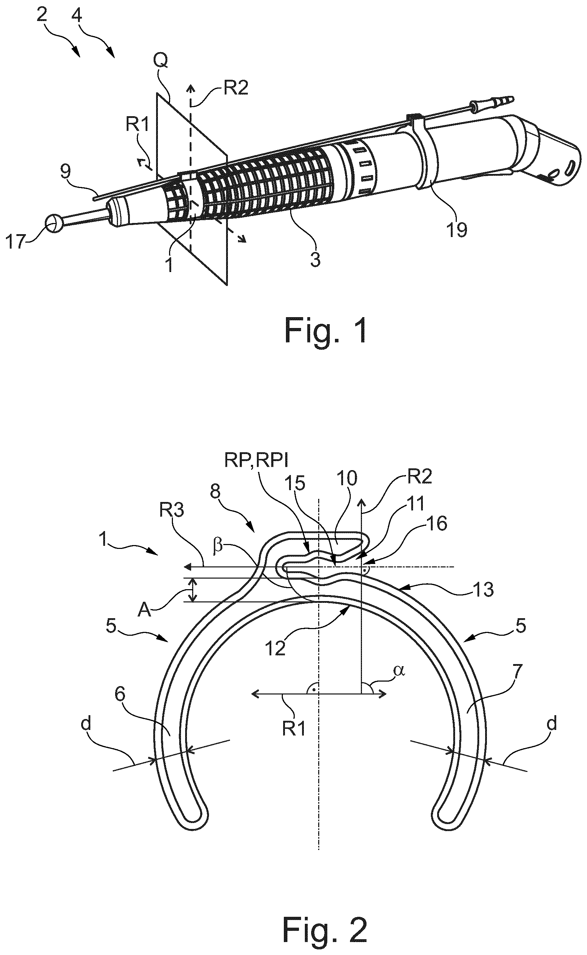

Fig.1 bis Fig.6b zeigen jeweils einen medizinischen Halteclip 1 zur lösbaren Montage an einem Medizinprodukt 2 insbesondere an einem medizinischen Handstück 3 einer medizinischen Vorrichtung 4 mit einem Montagebereich 5, der zwei Clipflügel 6, 7 aufweist, die gemeinsam eine C- oder U-Form ausbilden und die, in einer Halteclip-Querschnittsebene Q gesehen, elastisch längs einer ersten Richtung R1 biegbar sind, um die C- oder U-Form für eine Clipmontage des Halteclips 1 auf oder an dem Medizinprodukt 2 aufzuweiten. Der Halteclip 1 weist weiterhin einen Rast- oder Klemmbereich 8 auf, der dafür vorgesehen und ausgebildet ist, einen Schlauch oder ein Rohr 9 vorzugsweise parallel zum Medizinprodukt 2 klemmend sowie lösbar aufzunehmen.

Die zwei Clipflügel 6, 7 sind gleichartig biegbar. Im Wesentlichen weisen die Clipflügel 6, 7 benachbart des Klemmbereiches 8 eine zueinander spiegelsymmetrische Form auf. Denkbar wäre aber auch zueinander asymmetrische Clipflügel 6, 7 zu verwenden.

Der Rast- oder Klemmbereich 8 weist einen einstückig mit dem Montagebereich 5 ausgeformten oder an diesem befestigten Klemmflügel 10 auf, der zwischen sich und dem Rast- oder Klemmbereich 8 einen einseitig offenen Aufnahmespalt 11 ausbildet und der für ein Aufweiten des Aufnahmespalts 11, in der Halteclip-Querschnittsebene Q gesehen, längs einer zweiten Richtung R2 elastisch biegbar ist, welche in einem Winkel α ungleich 0° zur ersten Richtung R1 steht. Es ist vorzugsweise genau ein Klemmflügel 10 vorgesehen und vorhanden.

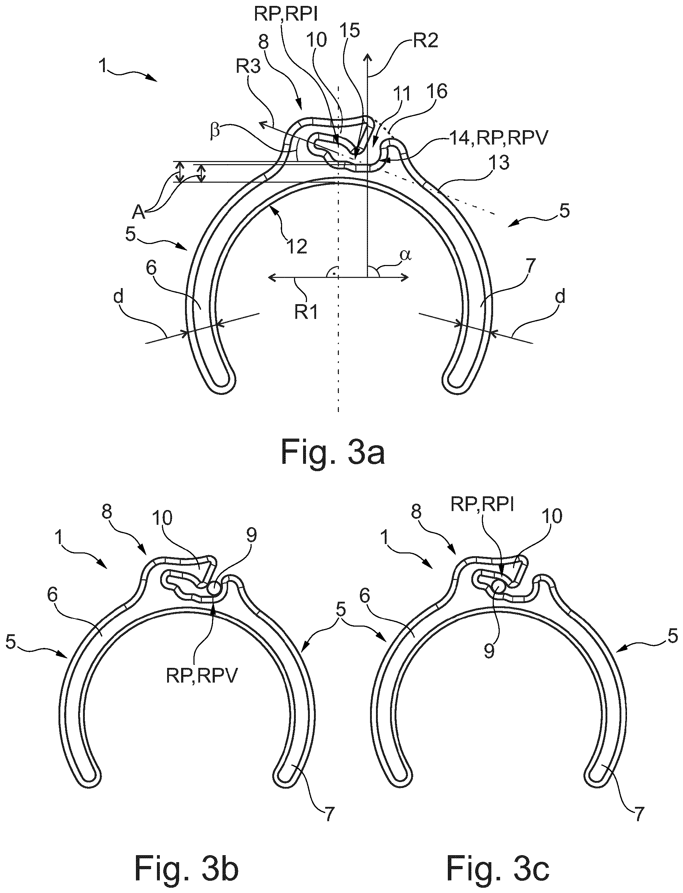

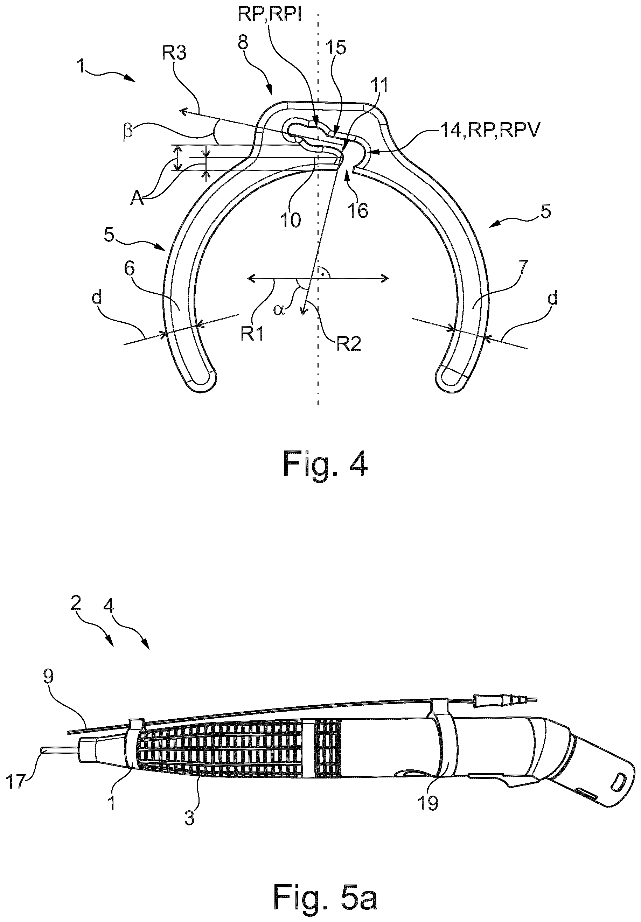

Die zweite Richtung R2 wird dabei wie folgt bestimmt. Es wird zunächst die Situation betrachtet, in welcher kein Schlauch oder Rohr 9 im Rast- oder Klemmbereich 8 aufgenommen ist. Aus dieser Konstruktionslage heraus beginnt sich der Klemmflügel 10 zu verbiegen, wenn der Schlauch oder das Rohr 9 durch eine Öffnung 16 des Aufnahmespalts 11 geführt wird. Das Verbiegen beginnt, wenn der erste Kontakt zwischen Schlauch oder Rohr 9 und dem Klemmflügel 10 erzeugt wird. Mit der zweiten Richtung R2, ist die Richtung gemeint, in welcher sich der Klemmflügel 10, insbesondere das auskragende Ende der neutralen Faser des Klemmflügels 10, zu Beginn des Verbiegens verbiegt. Je nach Ausprägung des Aufnahmespalts 11 kann es noch weitere Möglichkeiten geben, die zweite Richtung R2 zu bestimmen, wie zu Fig.3a und Fig.4 erläutert werden wird.

Der Halteclip 1 weist in einer Senkrechten zur Halteclip-Querschnittsebene Q bis auf abgerundete Kanten vorzugsweise gleichartige Querschnittsformen auf. Kleine Variationen dieser Querschnittformen zur Anordnung des Halteclips 1 an einem konischen Bereich des Medizinproduktes 2, insbesondere an einem zumindest teils konischen Handstücks 3, sind denkbar. Der Klemmflügel 10 ist vorzugsweise im Wesentlichen L-förmig ausgebildet, mit einem an die Clipflügel 6, 7 angrenzenden kurzen Schenkel, welcher in einen langen Schenkel übergeht, welcher das auskragende Ende des Klemmflügels 10 bildet.

Die Clipflügel 6, 7 definieren gemäß der ersten und zweiten Ausführungsform des medizinischen Halteclips 1 einen Innenseitenabschnitt 12, welcher dafür vorgesehen ist, mit dem Medizinprodukt 2 in vorzugsweise linienförmigen Klemmeingriff zu kommen und die Clipflügel 6, 7 definieren weiteren einen, von dem Innenseitenabschnitt 12 abgewandten Außenseitenabschnitt 13, welcher dafür vorgesehen ist, zusammen mit dem Klemmflügel 10 den Aufnahmespalt 11 zu bilden.

Gemäß der dritten Ausführungsform des medizinischen Halteclips 1 aus Fig.4 ist eine Öffnung 16 des Aufnahmespaltes 11 in dem Innenseitenabschnitt 12 der Clipflügel 6, 7 ausgebildet. Der Aufnahmespalt 11 bildet sich dann ausgehend von dieser Öffnung 16 in dem Rast- oder Klemmbereich 8 aus. Konkret wird der Aufnahmespalt 11 durch den Klemmflügel 10 und einen Abschnitt des Innenseitenabschnitts 12, der dafür vorgesehen ist nicht mit dem Medizinprodukt 2 in Klemmeingriff zu kommen, ausgebildet. Der Abschnitt ist zwischen Abschnitten des Innenseitenabschnitts 12 ausgebildet, die dafür vorgesehen sind, mit dem Medizinprodukt 2 in Klemmeingriff zu kommen. Der Klemmflügel 10 ist gemäß Fig.4 als Fortführung eines der Clipflügel 6, 7, nämlich des ersten Clipflügels 6, in Umfangsrichtung ausgeführt, so dass ein Radius des Innenseitenabschnittes 12 des Clipflügels 6, durch ein Aufbiegen des Klemmflügels 10 verkleinerbar ist.

Der Aufnahmespalt 11 erstreckt sich, insbesondere gemäß der ersten Ausführungsform des Halteclips 1 z.B. aus Fig.2, im Wesentlichen tangential zur C- oder U-Form der Clipflügel 6, 7. Hier erstreckt sich der Aufnahmespalt 11, in der Halteclip-Querschnittsebene Q gesehen, längs einer dritten Richtung R3 die im Wesentlichen parallel zur ersten Richtung liegt. Aber auch in den anderen Ausführungsformen des Halteclips 1 erstreckt sich jeweils ein bestimmter Abschnitt des Aufnahmespaltes 11 tangential zur C- oder U-Form der Clipflügel 6, 7.

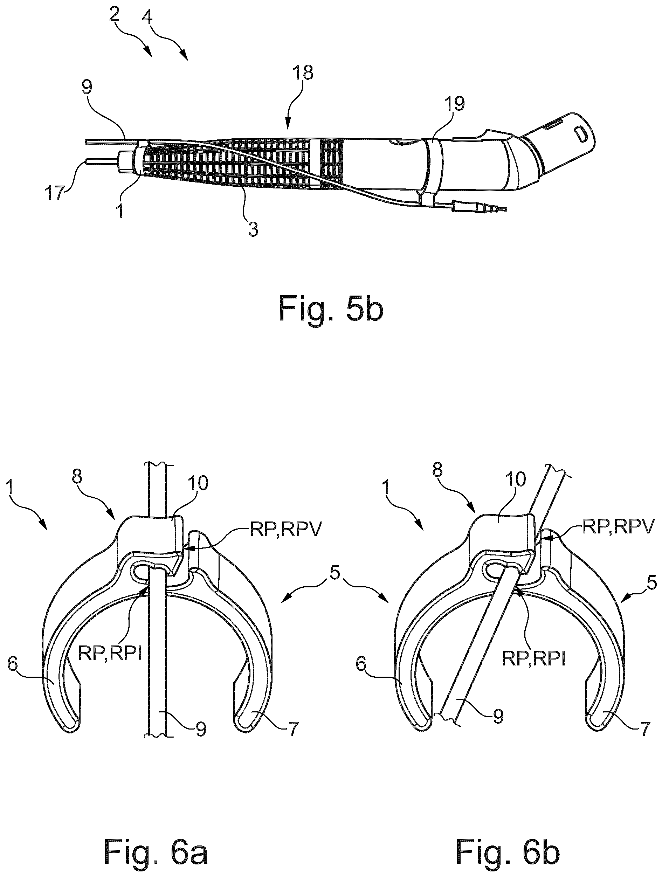

Der Aufnahmespalt 11 ist, in Auskragrichtung des Klemmflügels 10 gesehen, geradlinig, mäanderförmig, gekrümmt oder mit wenigstens einer knickförmigen Abbiegung 14 ausgebildet. Fig.2 zeigt die geradlinige Ausführung. Fig.3a, b und c, sowie Fig.4, Fig.6a und Fig.6b zeigen jeweils eine mäanderförmige, gekrümmte bzw. mit wenigstens einer knickförmigen Abbiegung 14 ausgebildete Ausführung. Die knickförmige Abbiegung 14 ist gemäß Fig.3a, b und c, sowie Fig.6a und Fig.6b mittels einer Erhebung an dem Außenseitenabschnitt 13 eines der Clipflügel, nämlich des zweiten Clipflügels 7, ausgebildet. Diese Erhebung ist in der Halteclip-Querschnittsebene Q gesehen nasen- oder wellenförmig ausgebildet und die Erhebung bildet senkrecht zur Halteclip-Querschnittsebene Q eine entsprechende Rippe.

Der Außenseitenabschnitt 13 der Clipflügel 6, 7 und/oder eine dem Außenseitenabschnitt 13 zugewandte Seite des Klemmflügels 10 ist, gemäß der ersten und zweiten Ausführungsform des medizinischen Halteclips 1, in Auskragrichtung des Klemmflügels 10 gesehen, gewellt oder gerippt, um zumindest eine, vorzugsweise eine Mehrzahl von, in Auskragrichtung des Klemmflügels 10 beabstandete einzelne Rastpositionen RP, RPI, RPV innerhalb des Aufnahmespalts 11 zu definieren.

Auch gemäß der dritten Ausführungsform des medizinischen Halteclips 1 aus Fig.4 weisen die den Aufnahmespalt 11 bildenden Oberflächen des Rast- oder Klemmbereichs 8 entsprechend gewellte oder gerippte Formen auf. Gemäß Fig.2 ist genau eine Rastposition RP, RPI realisiert. Gemäß Fig.3a, b und c, sowie Fig.4, Fig.6a und Fig.6b sind jeweils zwei Rastpositionen RP, RPI, RPV realisiert.

Der Aufnahmespalt 11 hat einen, an die in Auskragrichtung des Klemmflügels 10 gesehen, innerste Rastposition RPI unmittelbar angrenzenden Zufuhrabschnitt 15, durch welchen der Schlauch oder das Rohr 9 zur innersten Rastposition RPI hinführbar ist und der sich, in der Halteclip-Querschnittsebene Q gesehen, längs einer dritten Richtung R3 erstreckt, die in einem Winkel β von 65° bis 115°, vorzugsweise von 80° bis 100°, insbesondere gemäß Fig.2 von 90°, zu einer Senkrechten auf die erste Richtung R1 steht.

Der Aufnahmespalt 11 weist einen, von der innersten Rastposition RPI entgegen der Auskragrichtung des Klemmflügels 10 gesehenen, hier ausgebildeten innersten Abschnitt auf. Eine Länge dieses innersten Abschnittes definiert entgegen der Auskragrichtung des Klemmflügels 10 eine Länge eines mittels des Klemmflügels 10 ausgebildeten Hebels zur Erzeugung einer Klemmkraft auf einen in der innersten Rastposition RPI angeordneten Schlauch oder ein hier angeordnetes Rohr 9.

Ein Abstand A zwischen dem Innenseitenabschnitt 12 und dem Außenseitenabschnitt 13 der Clipflügel 6, 7 ist, gemäß der ersten und zweiten Ausführungsform des medizinischen Halteclips 1, in radialer Richtung zur Rastposition RP, RPI, RPV hin maximal so groß wie eine Dicke d der Clipflügel 6, 7 benachbart des Rast- oder Klemmbereichs 8. Analog sind auch gemäß Fig.4 entsprechend geringe Abstände A definierbar. Gemäß Fig.4 ist ein Abstand A zwischen dem Innenseitenabschnitt 12 und einer die Rastposition RP, RPI, RPV bildenden Oberfläche des Rast- oder Klemmbereiches 8 in radialer Richtung zur Rastposition RP, RPI, RPV hin maximal so groß wie eine Dicke d der Clipflügel 6, 7 benachbart des Rast- oder Klemmbereichs 8.

Die Clipflügel 6, 7 weisen benachbart dem Rast- oder Klemmbereich 8 bis auf eine Abrundung am jeweils auskragenden Ende und unter Umständen bis auf die Erhebung zur Ausbildung der knickförmigen Abbiegung 14 jeweils eine im Wesentlichen konstante Dicke d auf.

Der Aufnahmespalt 11 weist, gemäß Fig.3a, b und c, sowie Fig.4, Fig.6a und Fig.6b, zwischen seiner äußeren Öffnung 16 und der innersten Rastposition RPI eine Vor-Rastposition RPV auf, in welcher der Schlauch oder das Rohr 9 anordenbar, insbesondere einklemmbar ist.

Die Vor-Rastposition RPV ist über einen Aufnahmespaltabschnitt größter Krümmung, vorzugsweise mit knickförmiger Abbiegung 14 an die Aufnahmespaltöffnung 16 angeschlossen.

Die zweite Richtung R2 ist gemäß der zweiten und dritten Ausführungsform des Halteclips aus u.a. Fig.3a und Fig.4 auch wie folgt bestimmbar. Es kann hier nicht nur die Situation betrachtet werden, in welcher kein Schlauch oder Rohr 9 im Rast- oder Klemmbereich 8 aufgenommen ist, sondern auch die Situation, in welcher der Schlauch oder das Rohr 9 in der Vor-Rastposition RPV angeordnet ist. Mit dem in der Vor-Rastposition RPV angeordneten Schlauch oder Rohr 9 weist der Klemmflügel 10, wenn überhaupt, nur eine vernachlässigbar kleine Verbiegung gegenüber der Konstruktionslage ohne im Rast- oder Klemmbereich 8 aufgenommenen Schlauch oder Rohr 9 auf. Der Klemmflügel 10 beginnt sich dann insbesondere erneut zu Verbiegen, wenn der Schlauch oder das Rohr 9 aus der Vor-Rastposition RPV zur innersten Rastposition RPI hin bewegt wird. Mit der zweiten Richtung R2, ist dann auch die Richtung gemeint, in welcher sich der Klemmflügel 10, insbesondere das auskragende Ende der neutralen Faser des Klemmflügels 10, zu Beginn dieses insbesondere erneuten Verbiegens verbiegt.

Fig.1, Fig.5a und Fig.5b zeigen jeweils eine medizinische Vorrichtung 4 zur Aufnahme eines medizinischen Werkzeugs 17 mit einem Handstück 3, an welchem die medizinische Vorrichtung 4 von einem Mediziner führbar ist und mit zumindest einem medizinischen Halteclip 1, mittels welchem ein Schlauch oder ein Rohr 9 lösbar mit der medizinischen Vorrichtung 4, insbesondere dessen Handstück 3, verbindbar und/oder verbunden ist. Fig.1, Fig.5a und Fig.5b zeigen jeweils die verbundene Situation.

Der Schlauch oder das Rohr 9 ist mit einem weiteren, sogenannten Vor-Halteclip 19 in einem Bereich neben dem Handstück 3 mit der medizinischen Vorrichtung 4 verbunden.

Der Schlauch oder das Rohr 9 endet an einem vom Halteclip 1 auskragenden Ende, welches dem Werkzeug 17 zugewandt ist und welches auf einer dem Vor-Halteclip 19 abgewandten Seite des Halteclips 1 angeordnet ist. Der Vor-Halteclip 19 weist in einer Vor-Halteclip-Querschnittsebene gesehen zwei Klemmflügel auf, zwischen denen sich ein einseitig offener in radialer Richtung ausgerichteter Aufnahmespalt ausbildet, dessen äußere Öffnung tangential zu den Clipflügeln des Vor-Halteclips 19 liegt. Denkbar wäre aber auch anstelle des Vor-Halteclips 19 einen zweiten erfindungsgemäßen Halteclip zu verwenden.

Anhand von Fig.5a und Fig.5b wird nun ein Verfahren zum Werkzeugwechsel an der medizinischen Vorrichtung 4 beschrieben, in welchem das Handstück 3 mit dem daran lösbar verbundenen medizinischen Halteclip 1 zum Freigeben oder Arretieren des medizinischen Werkzeuges 17 um die Längsachse des Handstückes 3, von der in Fig.5a gezeigten, das Werkzeug 17 arretierenden Stellung zur in Fig.5b gezeigten das Werkzeug 17 freigegeben, verdrehten Stellung 18, gedreht wird, währenddessen der Schlauch oder das Rohr 9 in dem medizinischen Halteclip 1 eingeklemmt ist und eingeklemmt bleibt.

Der Schlauch oder das Rohr 9 wird beim Drehen des Handstückes 3 in seine verdrehte Stellung 18, in welcher das Werkzeug 17 freigegeben ist, zu einer Helix verformt. Die Relativposition des Vor-Halteclips 19 zur medizinischen Vorrichtung 4 wird während des Drehens nicht verändert.

Mittels Fig.6a und Fig.6b ist ein besonderer Vorteil der Ausführung des Halteclips 1 mit einer Vor-Rastposition RPV gezeigt. Fig.6a korrespondiert mit Fig.5a und zeigt einen in der innerste Rastposition RPI angeordneten Schlauch oder Rohr 9. Fig.6b korrespondiert mit Fig.5b und zeigt einen im Aufnahmespalt 11 schräg zur medizinischen Vorrichtung 4 angeordneten Schlauch oder ein hier entsprechend angeordnetes Rohr 9. Trotz dieser schrägen Anordnung ist der Schlauch oder das Rohr 9 sicher in dem Aufnahmespalt 11 eingeklemmt. Der Schlauch oder das Rohr 9 ist dann zu einen in einem ersten stirnseitigen Endbereich des Halteclips 1 im Bereich der innersten Rastposition RPI und auch im in einem dem ersten gegenüberliegenden zweiten stirnseitigen Endbereich des Halteclips 1 im Bereich der Vor-Rastposition RPV, insbesondere im Bereich der knickförmigen Abbiegung 14, jeweils am Rast- oder Klemmbereich 8 abgestützt. Weiterhin kontaktiert der Klemmflügel 10 den Schlauch oder das Rohr 9 in dieser Situation mittig zwischen den beiden Stirnseiten des Halteclips 1, so dass eine entsprechende Klemmkraft vom dann elastisch verbogenen Klemmflügel 10 auf den Schlauch oder das Rohr 9 wirkt.

Beim Zurückdrehen des Handstückes 3 von der verdrehten Stellung 18 in die das Werkzeug 17 arretierende Stellung rutscht der Schlauch oder das Rohr 9 vorzugsweise selbsttätig in die in Fig.6a gezeigte Position, nämlich die innerste Rastposition RPI, und ist dann wieder parallel zur medizinischen Vorrichtung 4 angeordnet.

### Bezugszeichenliste

- 1: medizinischer Halteclip
- 2: Medizinprodukt
- 3: medizinisches Handstück
- 4: medizinische Vorrichtung
- 5: Montagebereich
- 6: erster Clipflügel
- 7: zweiter Clipflügel
- 8: Rast- oder Klemmbereich
- 9: Schlauch oder Rohr
- 10: Klemmflügel
- 11: Aufnahmespalt
- 12: Innenseitenabschnitt
- 13: Außenseitenabschnitt
- 14: knickförmige Abbiegung
- 15: Zufuhrabschnitt des Aufnahmespalts 11
- 16: äußere Öffnung des Aufnahmespalts 11
- 17: medizinisches Werkzeug
- 18: verdrehte Stellung des Handstücks 3
- 19: Vor-Halteclip

- Q: Halteclip-Querschnittsebene
- R1: erste Richtung
- R2: zweite Richtung
- R3: dritte Richtung
- α: Winkel
- β: Winkel
- RP: Rastposition
- RPI: innerste Rastposition
- RPV: Vor-Rastposition
- A: Abstand
- d: Dicke

## Patentansprüche

1. Medizinischer Halteclip (1) zur lösbaren Montage an einem medizinischen Handstück (3) einer medizinischen Vorrichtung (4) mit
- einem Montagebereich (5), der zwei Clipflügel (6, 7) aufweist, die gemeinsam eine C- oder U-Form ausbilden und die, in einer Halteclip-Querschnittsebene (Q) gesehen, elastisch längs einer ersten Richtung (R1) biegbar sind, um die C- oder U-Form für eine Clipmontage des Halteclips (1) auf oder an dem Medizinprodukt (2) aufzuweiten und
- einem Rast- oder Klemmbereich (8), der einen einstückig mit dem Montagebereich (5) ausgeformten oder an diesem befestigten Klemmflügel (10) aufweist und dafür vorgesehen und ausgebildet ist, einen Schlauch oder ein Rohr (9) vorzugsweise parallel zum Medizinprodukt (2) klemmend sowie lösbar aufzunehmen, **dadurch gekennzeichnet, dass** der Klemmflügel (10) zwischen sich und dem Rast- oder Klemmbereich (8) oder dem Montagebereich (5) einen einseitig offenen Aufnahmespalt (11) ausbildet und für ein Aufweiten des Aufnahmespalts (11), in der Halteclip-Querschnittsebene (Q) gesehen, längs einer zweiten Richtung (R2) elastisch biegbar ist, welche in einem Winkel (α) ungleich 0° zur ersten Richtung (R1) steht.

2. Medizinischer Halteclip (1) nach Anspruch 1, **dadurch gekennzeichnet, dass** die Clipflügel (6, 7) einen Innenseitenabschnitt (12) definieren, welcher dafür vorgesehen ist, mit dem Medizinprodukt (2) in vorzugsweise linienförmigen Klemmeingriff zu kommen und die Clipflügel (6, 7) einen, von dem Innenseitenabschnitt (12) abgewandten Außenseitenabschnitt (13) definieren, wobei der Klemmflügel (10) den Aufnahmespalt (11) zusammen mit dem Innenseitenabschnitt (12) und/oder dem Außenseitenabschnitt (13) ausbildet.

3. Medizinischer Halteclip (1) nach Anspruch 1 oder 2, **dadurch gekennzeichnet, dass** sich der Aufnahmespalt (11) im Wesentlichen tangential zur C- oder U-Form der Clipflügel (6, 7) erstreckt.

4. Medizinischer Halteclip (1) nach Anspruch 1, 2 oder 3, **dadurch gekennzeichnet, dass** der Aufnahmespalt (11), in Auskragrichtung des Klemmflügels (10) gesehen, geradlinig, mäanderförmig, gekrümmt oder mit wenigstens einer knickförmigen Abbiegung (14) ausgebildet ist.

5. Medizinischer Halteclip (1) nach einem der vorstehenden Ansprüche 2 bis 4, **dadurch gekennzeichnet, dass** der Außenseitenabschnitt (13) der Clipflügel (6, 7) und/oder eine dem Außenseitenabschnitt (13) zugewandte Seite des Klemmflügels (10), in Auskragrichtung des Klemmflügels (10) gesehen, gewellt oder gerippt ist, um zumindest eine, vorzugsweise eine Mehrzahl von, in Auskragrichtung des Klemmflügels (10) beabstandete einzelne Rastpositionen (RP, RPI, RPV) innerhalb des Aufnahmespalts (11) zu definieren.

6. Medizinischer Halteclip (1) nach Anspruch 5, **dadurch gekennzeichnet, dass** der Aufnahmespalt (11) einen an die, in Auskragrichtung des Klemmflügels (10) gesehen, innerste Rastposition (RPI) unmittelbar angrenzenden Zufuhrabschnitt (15) hat, durch welchen der Schlauch oder das Rohr (9) zur innersten Rastposition (RPI) hinführbar ist und der sich, in der Halteclip-Querschnittsebene (Q) gesehen, längs einer dritten Richtung (R3) erstreckt, die in einem Winkel (β) von 65° bis 115°, vorzugsweise von 80° bis 100°, insbesondere von 90°, zu einer Senkrechten auf die erste Richtung (R1) steht.

7. Medizinischer Halteclip (1) nach einem der vorstehenden Ansprüche 5 bis 6, **dadurch gekennzeichnet, dass** ein Abstand (A) zwischen dem Innenseitenabschnitt (12) und dem Außenseitenabschnitt (13) der Clipflügel (6, 7) in radialer Richtung zur Rastposition (RP, RPI, RPV) hin maximal so groß ist wie eine Dicke (d) der Clipflügel (6, 7) benachbart des Rast- oder Klemmbereichs (8).

8. Medizinischer Halteclip (1) nach einem der vorangehenden Ansprüche 5, 6 oder 7, **dadurch gekennzeichnet, dass** der Aufnahmespalt (11) zwischen seiner äußeren Öffnung (16) und der innersten Rastposition (RPI) eine Vor-Rastposition (RPV) aufweist, in welcher der Schlauch oder das Rohr (9) anordenbar, insbesondere einklemmbar, ist.

9. Medizinischer Halteclip (1) nach Anspruch 8, **dadurch gekennzeichnet, dass** die Vor-Rastposition (RPV) über einen Aufnahmespaltabschnitt größter Krümmung, vorzugsweise mit knickförmiger Abbiegung (14) an die Aufnahmespaltöffnung (16) angeschlossen ist.

10. Medizinische Vorrichtung (4) zur Aufnahme eines medizinischen Werkzeugs (17) mit
- einem Handstück (3), mit welchem die medizinische Vorrichtung (4) von einem Mediziner führbar ist und mit
- zumindest einem medizinischen Halteclip (1) nach einem der vorrangehenden Ansprüche, mittels welchem ein Schlauch oder ein Rohr (9) lösbar mit der medizinischen Vorrichtung (4), insbesondere dessen Handstück (3), verbindbar und/oder verbunden ist.

11. Verfahren zum Werkzeugwechsel an einer medizinischen Vorrichtung (4) nach Anspruch 10, in welchem das Handstück (3) mit dem daran lösbar verbundenen medizinischen Halteclip (1) zum Freigeben oder Arretieren des medizinischen Werkzeuges (17) um die Längsachse des Handstückes (3) gedreht wird, währenddessen der Schlauch oder das Rohr (9) in dem medizinischen Halteclip (1) eingeklemmt ist und eingeklemmt bleibt.

12. Verfahren nach Anspruch 11, **dadurch gekennzeichnet, dass** der Schlauch oder das Rohr (9) beim Drehen des Handstückes (3) in seine verdrehte Stellung (18), in welcher das Werkzeug (17) freigegeben ist, zu einer Helix verformt wird.
